# EUROPÄISCHE PATENTSCHRIFT

(11) **EP 1 072 681 B1**
(45) Veröffentlichungstag und Bekanntmachung des Hinweises auf die Patenterteilung: **26.03.2003**
(21) Anmeldenummer: 00115190.1
(22) Anmeldetag: 13.07.2000
(51) Int. Cl.: C12N 15/12, C12N 15/10, C12N 15/11, C12N 15/85, C07K 14/47, C07K 16/18, C12Q 1/68, G01N 33/577, A01K 67/027

(54) **Regulatorisches Protein pKe#165 aus humanen Keratinozyten**
Human-keratinocytes regulator protein pKe#165
Protéine regulatrice pKe#165 des kératinocytes humaines

(30) Priorität: 23.07.1999 DE 19934603
(43) Veröffentlichungstag der Anmeldung: 31.01.2001
(73) Patentinhaber: Kramer, Michael, 64319 Pfungstadt (DE)
(72) Erfinder: Kramer, Michael, 64319 Pfungstadt (DE); Bechtel, Michael, 69198 Schriesheim (DE); Reinartz, Jeanette, 69121 Heidelberg (DE); Schäfer, Birgit, 69124 Heidelberg (DE); Wallich, Reinhard, 69118 Heidelberg (DE); Koch, Judith, 64732 Bad König (DE)
(74) Vertreter: Rudolph, Ulrike, Dr.

(56) Entgegenhaltungen:
- DATABASE EMBL/GENBANK/DDBJ [Online] EBI; 7. Juli 1999 (1999-07-07) "Mus musculus pleckstrin 2 mRNA" XP002157065
- JAEGER C: "IDENTIFICATION OF A NOVEL TRANSMEMBRANE-PROTEIN (PKE#192/MAP17) EXPRESSED BY HUMAN KERAMINOCYTES IN THE UPPER STRATUM GRANULOSUM OFTHE EPIDEMIS" JOURNAL OF INVESTIGATIVE DERMATOLOGY,US,NEW YORK, NY, Bd. 112, Nr. 4, 1999, Seite 574 XP000909987 ISSN: 0022-202X
- DELLAMBRA, E. ET AL.: "Stratifin, a keratinocyte specific 14-3-3 protein, harbors a pleckstrin homology (PH) domain and enhances protein kinase C activity" J. CELL SCI., Bd. 108, 1995, Seiten 3569-3579, XP000971860

## Beschreibung

Die Erfindung betrifft ein isoliertes Polypeptid, das natürlicherweise in humanen epidermalen Keratinozyten vorkommt. Sie betrifft außerdem eine isolierte Nukleinsäure, die ein solches Polypeptid kodiert, sowie die Verwendung dieses Polypeptids und dieser Nukleinsäure für nachweisende Zwecke und für Reagenzien, insbesondere rekombinante Vektormoleküle und Antikörper.

Nach dem gegenwärtigen Stand der Technik werden in der Dermatotherapie zur Beeinflussung epidermaler Störungen wie z.B. der Autoimmundermatosen "Pemphigus vulgaris" und "Bullöses Pemphigoid" im wesentlichen Medikamente mit breitem Wirkungsspektrum eingesetzt, insbesondere lokal bzw. systemisch applizierte Glukokortikoide, Vitamin-A-Säure-Derivate, Antimetabolite und Zytostatika, oder es wird mit mehr oder weniger unspezifischen Maßnahmen wie z.B. der sog. "Farbstofftherapie" oder der "Lichttherapie" behandelt. Die bekannten Wirkstoffe bzw. Maßnahmen haben jedoch allesamt den Nachteil, daß sie wenig spezifisch sind und damit naturgemäß zahlreiche Nebenwirkungen hervorrufen.

Die Bereitstellung spezifischerer Wirkstoffe scheiterte bislang an dem in der Dermatologie seit langem bestehenden grundsätzlichen Problem, daß die Zahl der zellulären Zielmoleküle, im folgenden allgemein als Zielstrukturen ("Targets") benannt, die als Angriffspunkt für eine (spezifische) Beeinflussung des zellulären Stoffwechsels - insbesondere unter medizinischen oder auch kosmetischen Gesichtspunkten - dienen könnten, in epidermalen Keratinozyten eng begrenzt ist.

Aufgabe der vorliegenden Erfindung ist es deshalb, neue Zielstrukturen in epidermalen Keratinozyten bereitzustellen, die als Angriffspunkt für Diagnostika, Therapeutika, Kosmetika oder allgemein für die Beeinflussung des zellulären Stoffwechsels dienen können.

Eine Lösung dieser Aufgabe besteht in der Bereitstellung eines Polypeptids bzw. Proteins der eingangs genannten Art, das die im Sequenzprotokoll SEQ ID NO: 2 dargestellte Aminosäuresequenz aufweist, natürlicherweise in humanen epidermalen Keratinozyten vorkommt und im aktivierten, durch eine erhöhte Expression der Aktivierungsmarker uPA und uPA-R gekennzeichneten Zustand dieser Keratinozyten aufreguliert, nämlich verstärkt exprimiert wird Das Polypeptid mit der Aminosäuresequenz gemäß **SEQ ID NO: 2** wird im folgenden auch mit Protein "pKe#165" bezeichnet.

Eine weitere Lösung der genannten Aufgabe besteht in der Bereitstellung einer isolierten Nukleinsäure, die entweder die im Sequenzprotokoll SEQ ID NO: 1 dargestellte Nukleotidsequenz oder eine ganz oder teilweise mit dieser unter stringenten Bedingungen hybridisierende Nukleotidsequenz aufweist, und die ein Polypeptid gemäß Anspruch 1 codiert.
Die erfindungsgemäßen Nukleotidsequenzen sind außerdem dadurch gekennzeichnet, daß sie entweder direkt oder in Form der durch sie codierten Polypeptide zur Beeinflussung der Zellproliferation, Zelladhäsion, Zellmigration, Zellmorphogenese und/oder Zelldifferenzierung - insbesondere von Keratinozyten - geeignet sind.

Der Begriff "hybridisieren" bzw. "Hybridisierung" gemäß vorliegender Erfindung wird wie bei *Sambrook et al., Molecular Cloning, A Laboratory Manual, Cold Spring Harbor*, *Laboratory Press, 1989, 1.101 bis 1.104* verwendetund bezieht sich auf die im Stand der Technik bekannten Hybridisierungsverfahren unter üblichen, insbesondere unter hoch stringenten Hybridisierungsbedingungen. Nach *Sambrook et al.* spricht man von einer Hybridisierung unter stringenten Bedingungen, wenn nach Waschen für eine Stunde mit 1 x SSC und 0,1% SDS, vorzugsweise mit. niedriger konzentriertem SSC, insbesondere 0,2 x SSC, bei einer Temperatur von wenigstens 55°C, vorzugsweise 62°C und besonders bevorzugt 68°C noch ein positives Hybridisierungssignal beobachtet wird. Jede Nukleotidsequenz, die unter derartigen Waschbedingungen mit einer Nukleotidsequenz gemäß **SEQ ID NO:** 1 oder mit einer der Sequenz gemäß **SEQ ID NO: 1** im Rahmen der Degeneration des genetischen Codes entsprechenden Nukleotidsequenz hybridisiert, gehört zum Gegenstand der vorliegenden Erfindung. Im konketen Einzelfall wählt der Fachmann die konkreten Hybridisierungsparameter anhand der eingesetzten Nukleotidsequenz und seines allgemeinen Fachwissens (vgl.: *Current Protocols in Molecular Biology, Vol. 1, 1997, John wiley & Sons Inc., Suppl. 37, Chapter 4.9.14*).

Die erfindungsgemäße(n) Nukleinsäure(n) kann (können) sowohl aus einer natürlichen Quelle als auch synthetisch oder halbsynthetisch gewonnen werden. In der Praxis hat sich besonders die Ausführung als cDNA bewährt.

Das Polypeptid, das die Aminosäuresequenz gemäß **SEQ ID NO: 2** aufweist und von der im Sequenzprotokoll **SEQ ID NO: 1** dargestellten Nukleinsäure kodiert wird, und das im folgenden als Protein "pKe#165" bezeichnet ist, wird in humanen epidermalen Keratinozyten aufreguliert, nämlich verstärkt exprimiert (produziert) und auf einem im Vergleich zum Ausgangszustand signifikant höheren Konzentrationsspiegel gehalten, wenn sich diese Zellen im "aktivierten" Zustand befinden, d.h. unter anderem im Zustand der Proliferation und/oder Migration, z.B. nach einer unfallbedingten Hautverletzung oder bei den autoimmunologisch ausgelösten bullösen Dermatosen "Pemphigus vulgaris" (ausgelöst durch Autoantikörper gegen Desmosomen) und "Bullöses Pemphigoid" (ausgelöst durch Autoantikörper gegen Hemidesmosomen). Der aktivierte Zustand der humanen epidermalen Keratinozyten äußert sich auch in einer im Vergleich zum Ruhezustand (Ausgangszustand) erhöhten Expression der bekannten Aktivierungsmarker uPA (Urokinase-Typ Plasminogenaktivator) und uPA-R (Rezeptor für Urokinase-Typ Plasminogenaktivator) und kann anhand dieser Marker qualitativ und quantitativ nachgewiesen werden (vgl.: *Schäfer, et al., 1996: Dispase-mediated basal detachment of cultured keratinocytes induces urokinase-type plasminogen activator (uPA) and its receptor (uPA-R, CD87), Exp. Cell Res. 228, pp.* 246 - 253).

Das Protein pKe#165 weist eine sog. Pleckstrin-Domäne auf. Hierbei handelt es sich um eine Bindungsstelle für Phosphatidyl-Inositol-Diphosphat, Phosphatidyl-Inositol-Triphosphat und die beta/gamma-Untereinheit von trimeren G-Proteinen (vgl.: *Lemmon, M.A. et. al., 1995: Specific and highaffinity binding of inositol phosphates to an isolated pleckstrin homology domain, Proc. Natl. Acad. Sci. USA 92, 10472-10476; Ferguson, K.M. et al., 1995: Structure of the high affinity complex of inositol trisphosphate with a phospholipase C pleckstrin homology domain, Cell 83. 1037-1046; Harlan, J.E. et. al., 1994: Pleckstrin homology domains bind to phosphatidylinositol-4,5-bis-phosphate, Nature 371, 168-170; Touhara, K. et al., 1994: Binding of G protein βγ-subunits to pleckstrin homology domains, J. Biol. Chem., 269, 10217-10220*). Darüber hinaus weist das Molekül eine Reihe möglicher Phosphorylierungsstellen auf. Diese Motive sind ein Indiz dafür, daß das Protein in Signaltransduktionsabläufe eingebunden ist.

Mit der (isolierten) Bereitstellung des Proteins "pKe#165", nämlich mit der Beschreibung von Nukleotidsequenzen, die dieses Protein kodieren, und mit der Angabe (einer) seiner Aminosäuresequenz(en) ist es möglich, den Stoffwechsel physiologisch aktiver bzw. aktivierter Keratinozyten - und selbstverständlich auch anderer das Protein "pKe#165" exprimierende Zellen - gezielt zu beeinflussen, insbesondere zu Zwecken der medizinischen Therapie und kosmetischen Behandlung.

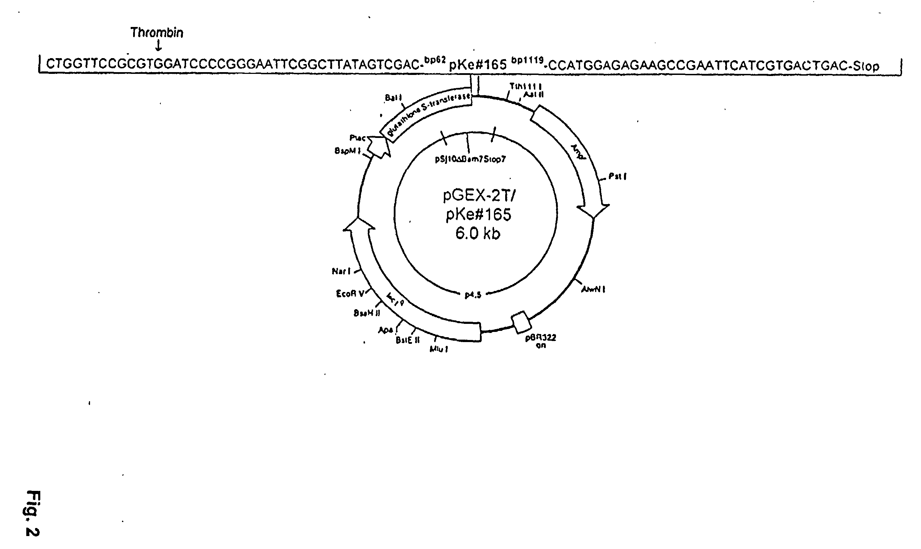

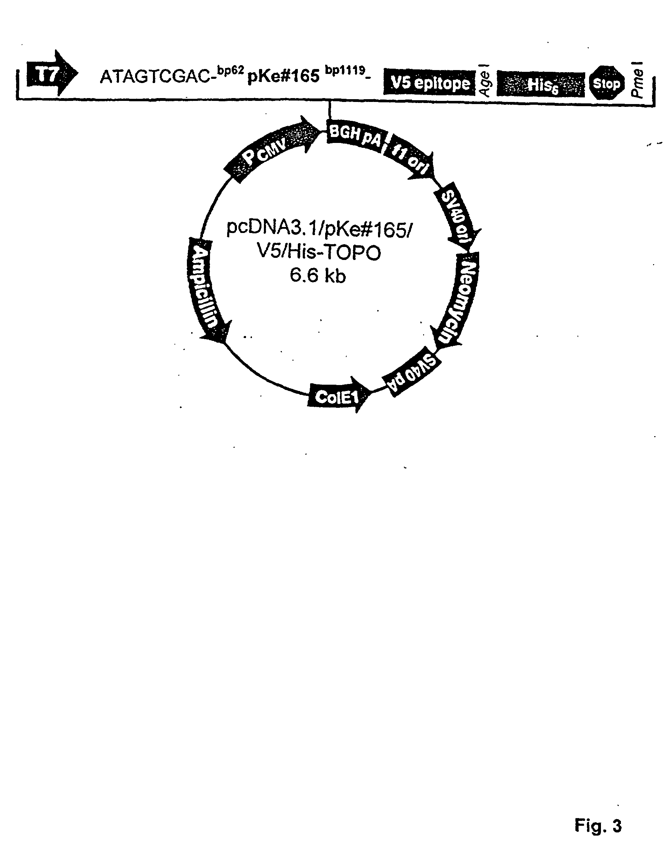

Die Erfindung betrifft desweiteren rekombinante DNS-Vektormoleküle, die eine erfindungsgemäße Nukleinsäure umfassen, und die die Fähigkeit zur Expression des Polypeptids gemäß Anspruch 1(des Proteins pKe#165) in einer prokaryontischen oder eukaryontischen Zelle aufweisen. Bei diesen DNS-Vektormolekülen handelt es sich vorzugsweise um Abkömmlinge des Plasmids pUEX-1 und/oder des Plasmids pGEX-2T und/oder des Plasmids pcDNA3.1, da sich diese Vektoren in der Praxis als sehr gut geeignet erwiesen haben. Besonders bevorzugt sind das Vektorkonstrukt pGEX-2T/pKe#165 gemäß dem in Fig. 2 offenbarten Vektorprotokoll und das Vektorkonstrukt pcDNA3.1/pKe#165/V5/His-TOPO gemäß dem in Fig. 3 offenbarten Vektorprotokoll. Als eukaryontische Zelle kommen insbesondere Zellen aus Zellkulturen, z.B. Cos-Zellen, in Betracht.

Die Erfindung umfaßt deshalb auch transformierte Wirtszellen, die ein Vektormolekül nach einem der Ansprüche 5 bis 7 enthalten, sowie transformierte Wirtszellen, die eine Nukleinsäure nach einem der Ansprüche 2 bis 4 enthalten, wobei diese Nukleinsäure mit einem aktivierbaren Promotor gekoppelt ist und dadurch gekennzeichnet ist, daß der Promotor der Zytokeratin-14-Promotor und die Wirtszelle ein Keratinozyt ist, oder daß der Promotor der CMV-Promotor und die Wirtszelle eine Cos-Zelle ist.

Derartige Wirtszellen besitzen die Fähigkeit zur Expression des in humanen Keratinozyten vorkommenden und im aktivierten Zustand der Keratinozyten verstärkt exprimierten Proteins "pKe#165",.

Die erfindungsgemäßen Transfektanten ermöglichen Forschungs- und Entwicklungsarbeiten zum Zweck der weitergehenden Aufklärung der durch das Protein "pKe#165" induzierten Veränderungen der Zellmorphologie und zellulären Basisfunktionen wie Proliferation, Adhäsion, Migration und Differenzierung, insbesondere im Hinblick auf die Beantwortung der Frage, ob das Protein "pKe#165" selbst eine "pathogene" Aktivität besitzt.

Gegenstand der vorliegenden Erfindung ist außerdem ein Reagenz zum indirekten Nachweis des in humanen Keratinozyten vorkommenden und im aktivierten Zustand der Keratinozyten verstärkt exprimierten Proteins mit der Aminosäuresequenz gemäß SEQ ID NO: 2 wobei dieses Reagenz dadurch charakterisiert ist, daß es wenigstens eine erfindungsgemäße Nukleinsäure umfaßt. "Zum indirekten Nachweis" bedeutet in diesem Zusammenhang, daß tatsächlich die das Protein kodierende mRNA direkt nachgewiesen wird - und somit das Protein nur indirekt (vermittels dieser mRNA).

Das Protein "pKe#165" mit der im Sequenzprotokoll **SEQ ID NO: 2** dargestellten Aminosäuresequenz bieten vielfältige Anwendungsmöglichkeiten auf dem Gebiet der dermatologischen Forschung und Entwicklung. Insbesondere können Antikörper gegen dieses Polypeptid hergestellt werden, die dann mit entsprechender Modifikation entweder als Diagnostika oder als Therapeutika oder auch als Kosmetika ("cosmeceuticals") einsetzbar sind.

Die Erfindung umfaßt folglich auch die Verwendung eines solchen Proteins bzw. Polypeptids zur Herstellung eines (monoklonalen, polyklonalen oder rekombinanten) Antikörpers gegen dieses Polypeptid und den besagten Antikörper selbst.

Eine technisch und wirtschaftlich bedeutende Einsatzmöglichkeit eines erfindungsgemäßen Polypeptids und ebenso einer erfindungsgemäßen Nukleinsäure besteht nicht zuletzt auch darin, daß mit Hilfe eines solchen Moleküls in einem "Screening"-Verfahren aus einer sehr großen Anzahl bereitstehender Stoffe solche herausselektiert werden können, die spezifisch an die betreffende Nukleinsäure oder das betreffende Polypeptid binden. Diese Stoffe können dann als Ausgangsmaterial (Leitstruktur) für die Entwicklung pharmakologisch einsetzbarer Substanzen dienen und bieten damit die Voraussetzungen für die Entwicklung alternativer Pharmazeutika zur Diagnose und Therapie, insbesondere der eingangs erwähnten dermatologischen Erkrankungen und/oder anderer Erkrankungen, bei denen das Protein "pKe#165" eine Rolle spielt.

Im Hinblick darauf betrifft die Erfindung auch die Verwendung eines erfindungsgemäßen Polypeptids oder einer erfindungsgemäßen Nukleinsäure in einem Screeningverfahren zur Identifizierung pharmakologisch einsetzbarer Substanzen, die an das Polypeptid bzw. die Nukleinsäure binden und dadurch dessen bzw. deren Funktion und/oder Expression beeinflussen, insbesondere inhibierend oder aktivierend wirken.
- Fig. 1:: einen rt-PCR-Nachweis von "pKe#165"-spezifischer mRNA
- Fig. 2:: das Vektorkonstrukt pGEX-2T/pKe#165 für bakterielle Expressionssysteme
- Fig. 3:: das Vektorkonstrukt pcDNA 3.1/pKe#165/V5/His-TOPO für eukaryontische Expressionssysteme
- Fig. 4:: einen Immunoblot-Nachweis (Western-Blot) von rekombinantem pKe#165 Protein in E.coli-Zellen nach Transfektion mit dem Vektorkonstrukt pGEX-2T/pKe#165
- Fig. 5:: einen Immunoblot-Nachweis von rekombinantem pKe#165 Protein in Cos-Zellen nach Transfektion mit dem Vektorkonstrukt pcDNA 3.1/pKe#165/V5/His-TOPO und Anwendung eines anti-His-Antikörpers
- Fig. 6:: einen Immunoblot-Nachweis von rekombinantem pKe#165 Protein in Cos-Zellen nach Transfektion mit dem Vektorkonstrukt pcDNA 3.1/pKe#165/V5/His-TOPO und Anwendung eines Maus-anti-pKe#165-Antikörpers
- Fig. 7:: einen "Sandwich"-ELISA-Test unter Verwendung von Antikörpern, die gegen das Protein pKe#165 gerichtet sind.
- Fig. 8:: einen Immunfluoreszenztest unter Verwendung von Kaninchen "anti-pKe#165 IgG" auf Gefrierschnitten von Normalhaut und läsionaler Haut von bullösem Pemphigoid
- Fig. 9:: Keratinozyten (HaCaT-Zellen) nach Behandlung mit Kontroll-Oligonukleotiden (A) und mit pKe#165-spezifischen Antisense-Oligonuleotiden (B)

### Beispiel 1: Herstellung des Proteins "pKe#165"

### A) Gewinnung und Herstellung eines Polynukleotids, das das Protein "pKe#165" kodiert

Als Polynukleotidquelle dienten humane epidermale Keratinozyten einer Zellkultur bzw. eines Zellkulturmodells, das in der Publikation von *Schäfer B.M. et al., 1996: Dispase-mediated basal detachment of cultured* *keratinocytes induces urokinase-type plasminogen activator (uPA) and its receptor (uPA-R, CD87), Exp. Cell Res. 228, pp. 246-253,* ausführlich beschrieben ist. Diese Zellkultur bzw. dieses Zellkulturmodell zeichnet sich dadurch aus, daß sie/es erlaubt, Keratinozyten durch enzymatische Zerstörung der Zell/Matrix-Kontakte, beispielsweise durch eine Dispaseinduzierte Ablösung der Keratinozyten von der Kulturmatrix, vom ruhenden [uPA⁻/uPA-R⁻] in den aktivierten [uPA⁺/uPA-R⁺] Zustand zu überführen. Die Induktion des aktivierten Zustands ist reversibel: die (erneute) Ausbildung eines konfluenten (= maximal dicht gewachsenen), mehrschichtigen Zellverbands aus differenzierten Keratinozyten führt zur Abregulierung von uPA und uPA-R, d.h. zur Drosselung der Produktion und Einstellung auf einem niedrigeren Konzentrationsspiegel (siehe dazu die Publikation von *Schäfer B.M. et al., 1996: Differential expression of urokinase-type plasminogen activator (uPA), its receptor (uPA-R), and inhibitor type-2 (PAI-2) during differentiation of keratinocytes in an organotypic coculture System. Exp. Cell Res. 220, pp. 415-423*).

Die Zellen dieser Zellkultur bzw. dieses Zellkulturmodells werden im folgenden auch als NHEK (= "normale humane epidermale Keratinozyten") bezeichnet.

Für die Bereitstellung der Zellkultur bzw. des Zellkulturmodells wurden folgende Maßnahmen durchgeführt: Mittels Hautbiopsie erhaltene NHEK wurden über Nacht bei 4°C trypsiniert und anschließend nach der "Feeder Layer"-Technik von *J.G. Rheinwald und H. Green (1975, Serial cultivation of strains of human epidermal keratinocytes: the formation of keratinizing colonies from single cells, Cell 6, pp. 331-334*) in Petrischalen oder in 175 cm² Kulturflaschen für die Dauer von 8 Tagen in Dulbecco's modified Eagle's Medium (DMEM) mit einem Gehalt von 10 Vol.-% fötalem Kälberserum (FCS) und Zusätzen an Adeninhemisulfat, Insulin, Transferrin, Trijodthyronin, Hydrocortison, Forskolin, epidermalem Wachstumsfaktor (EGF) und Antibiotika (Penicillin, Streptomycin und Gentamycin) unter Differenzierungsbedingungen, insbesondere erhöhten Kalziumspiegeln, kultiviert (37°C, 7% CO₂). Die Kultivierung erfolgte damit gemäß herkömmlicher und im Stand der Technik geläufigen Bedingungen. Unter diesen Bedingungen bilden Keratinozyten konfluente zwei- bis dreischichtige sog. "Epidermisäquivalente" oder Keratinozyten-"Sheets" aus.

Diese Epidermisäquivalente bzw. Keratinozytensheets wurden durch eine 30-minütige Behandlung mit Dispase II (2,4 mg/ml in DMEM ohne FCS) von der Kulturmatrix abgelöst, zweimal in DMEM gewaschen und anschließend für die Dauer von 4 - 8 Stunden in komplettem, konditioniertem DMEM inkubiert. Die Inkubation in konditioniertem DMEM erfolgte, um den Einfluß von frischem FCS auszuschließen. Während der Inkubation fand in diesen flotierenden Keratinozytensheets eine Aufregulierung der bekannten Aktivierungsmarker uPA und uPA-R sowie des hierin erstmals beschriebenen Proteins pKe#165 statt. Die uPA/uPA-R-Aufregulierung war mittels bekannter Techniken wie Enzyme-Linked-lmmunosorbent Assay (ELISA), in situ-Hybridisierung und Immunfluoreszenz nachweisbar. Aus den inkubierten Zellen wurde mittels der im Stand der Technik bekannten Guanidinium-Thiocyanat-Phenol-Chloroform-Extraktionsmethode (vgl.: *Chromczynski P. and Sacchi N., 1986: Single-step method of RNA isolation by acid guanidinium thiocyanate-phenol-chloroform extraction. Anal. Biochem. 162: pp. 156-159*) die gesamte RNA gewonnen (Kit "RNA-Clean" der Firma AGS, Heidelberg). Aus der Gesamt-RNA wurde die mRNA mittels Bindung an Poly-T-beschichtete Kügelchen isoliert. Diese mRNA diente als Ausgangsmaterial für den nächstfolgenden Verfahrensschritt der Subtraktionsklonierung.

Für den Einsatz in Kontrollversuchen bzw. für Vergleichspräparate wurde mRNA von adhärenten Keratinozytensheets isoliert, und zwar nach dem gleichen Verfahrensmuster wie vorstehend beschrieben, ausgenommen der Abweichung, daß für die Dauer der Dispasebehandlung zusätzlich zu der Dispase ein Dispasehemmer, z.B. Phosphoramidon (100 µg/ml), appliziert wurde.

Nach dem Prinzip der Subtraktionsklonierung wurde eine Genbank erstellt, die vorzugsweise cDNA der dyshäsionsinduzierten Gene enthielt, d.h. solcher Gene, die nach Ablösung der Keratinozytensheets vermehrt in diesen (bzw. deren Zellen) exprimiert wurden. Zu diesem Zweck wurde die aus den Zellen der adhärenten Keratinozytensheets gewonnene mRNA erneut an poly-T-beschichtete Kügelchen gebunden, auf diesen in einzelsträngige cDNA umgeschrieben und anschließend gegen die mRNA abgelöster, d.h. nicht-adhärenter Keratinozytensheets hybridisiert. Diejenigen mRNA-Moleküle, die lediglich im nicht-adhärenten Zustand, d.h. nach Dyshäsion exprimiert wurden und infolgedessen keinen Hybridisierungspartner fanden, verblieben im Überstand. Sie wurden in cDNA umgeschrieben und in den Klonierungsvektor pUEX-1 kloniert.

Die daraus resultierende Genbank wurde zwecks Überprüfung anschließend noch einem Southernblot-Verfahren mit [³²P]-markierter cDNA adhärenter und nicht-adhärenter Keratinozytensheets unterworfen. Diejenige cDNA oder vielmehr die sie enthaltenden Wirtszellklone - hier des E. coli Stamms MC1061 -, die nach Dyshäsion eine deutliche Aufregulation zeigten, wurden anschließend über Nacht bei 30°C unter üblichen Kulturbedingungen kultiviert bzw. vermehrt. Aus diesen E. coli-Klonen wurde die Plasmid-DNA (pUEX1-cDNA) herauspräpariert, und die aus dem pUEX1-Vektor herausgeschnittenen cDNA-Fragmente wurden mittels Random-priming [³²P]-markiert. Die markierte cDNA wurde als Sonde in Northemblots mit RNA aus adhärenten und nicht-adhärenten Keratinozytensheets eingesetzt. Die Klone, die cDNA enthielten, die bei Verwendung als Sonde im Northernblot-Verfahren kein oder nur ein geringes Signal mit der RNA adhärenter Keratinozyten, dagegen ein deutliches Signal mit RNA nicht-adhärenter Keratinozytensheets erkennen ließen, wurden für den nachfolgenden Verfahrensabschnitt der Sequenzierung ausgewählt.

Bei der Sequenzierung der betreffenden Klone mittels des "nichtradioaktiven Cycle-Sequencing", das eine Modifikation der Sequenzierungsmethode nach Sanger (*F. Sanger et al., 1977: DNA sequencing with chain terminating inhibitors, Proc Natl Acad Sci USA 74: 5463-5467*) darstellt und mittlerweile eine dem Stand der Technik geläufige Methode ist, wurde unter anderem das Gen mit der Nukleotidsequenz gemäß Sequenzprotokoll **SEQ ID NO: 1** erhalten. Das Gen mit der Nukleotidsequenz gemäß Protokoll **SEQ ID NO: 1** und das zugehörige Protein erhielten die Bezeichnung "pKe#165".

Nähere Untersuchungen der zu dem Gen pKe#165 gehörigen, d.h. pKe#165-spezifischen mRNA (aus abgelösten, d.h. nicht-adhärenten Keratinozytensheets) lieferten die Informationen, daß diese mRNA eine Größe von etwa 2,6 kb aufweist. Die Nukleotidsequenz gemäß **SEQ ID NO:1** enthält am 3'Ende an Position 1107-1109 ein Stopcodon, das den mutmaßlichen Ort des Transkriptionsendes vorgibt. An Position 1435-1439, genau 14 Nukleinsäuren vor der poly-A-Site befindet sich eine sog. "Polyadenylation site".

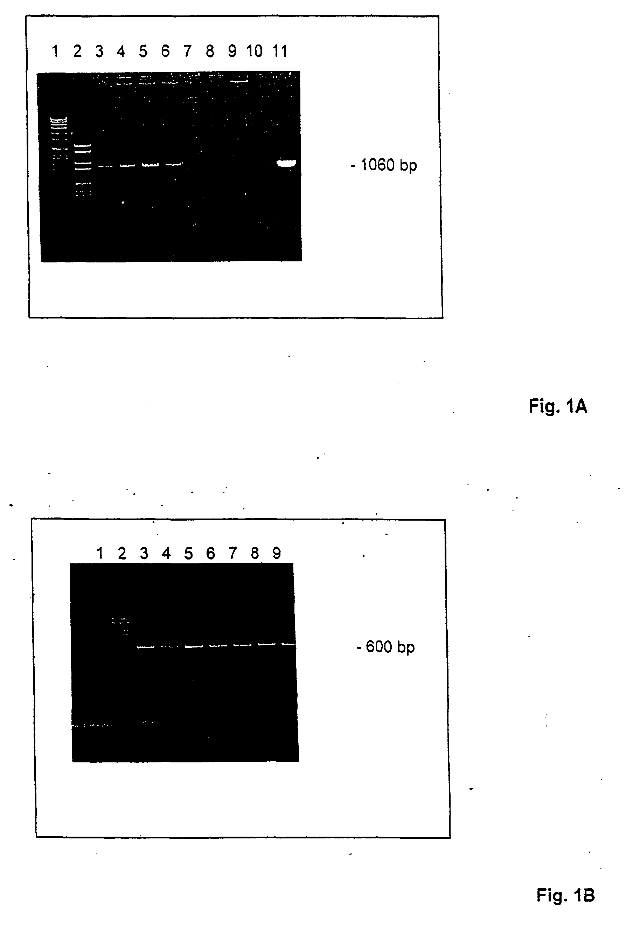

Mit Hilfe der Polymerasekettenreaktion konnte gezeigt werden, daß die pKe#165-spezifische mRNA nach Dispase-induzierter Ablösung der NHEK eine Aufregulation erfährt. In **Fig. 1** ist das Ergebnis einer Polymerasekettenreaktion nach reverser Transkription (rt-PCR) der mRNA in cDNA und Amplifikation mit pKe#165-spezifischen Oligonukleotid-Primern dargestellt. Dieses Ergebnis beinhaltet die Aussage, daß direkt nach der Ablösung der NHEK nur wenig pKe#165-mRNA vorhanden oder jedenfalls nachweisbar war, daß aber bereits 2 Stunden später eine deutliche Aufregulation festgestellt werden konnte.

### B) Ableitung der Aminosäurenabfolge und Charakterisierung des Proteins "pKe#165" anhand des dafür kodierenden Polynukleotids

Anhand des genetischen Codes der "pKe#165"-cDNA wurde mit Hilfe eines computergestützten Verfahrens (Programm "HUSAR" [= Heidelberg Unix Sequence Analysis Ressources] Version 4.0, Deutsches Krebsforschungszentrum Heidelberg, 1997) von der Nukleotidsequenz gemäß Sequenzprotokoll **SEQ ID NO: 1** eine Aminosäuresequenz abgeleitet, die im Sequenzprotokoll **SEQ ID NO: 2** dargestellt ist. Die strukturelle Analyse dieser Aminosäuresequenz gemäß Sequenzprotokoll **SEQ ID NO: 2** mit eben diesem Programm lieferte folgende Informationen:

Aus der Aminosäurezusammensetzung des Proteins pKe#165 errechnet sich ein Molekulargewicht von 39 980 Da mit einem isoelektrischen Punkt von pH 10,2.

### Beispiel 2: Nachweis "pKe#165"-spezifischer mRNA in Zellen mittels reverser Polymerasekettenreaktion

Mittels der Polymerasekettenreaktion nach reverser Transkription (rt-PCR) wurde pKe#165-spezifische mRNA in Zellen (NHEK) von Keratinozytensheets nach Dispasebehandlung und in HaCaT-Zellen bzw. HMEC-Zellen nachgewiesen. Hierfür wurde RNA aus Zellen von Keratinozytensheets nach Dispasebehandlung und unterschiedlich langer weiterer Inkubationszeit und aus HaCaT-Zellen bzw. HMEC-Zellen jeweils mit Standardmethoden (Guanidinium-Thiocyanat-Phenol-Chloroform-Extraktionsmethode) isoliert und nach Standardmethoden in cDNA umgeschrieben. Diese cDNA wurde einer PCR unterzogen, bei der aus der pKe#165-spezifischen cDNA ein Teilfragment von 1060 Bp amplifiziert wurde. Als Primer-Paar wurde eine Kombination aus pKe#165-forward-5a (ATAGTCGACATGGAGGACGGCGTGCTCAAG) und pKe#165-reverse-3a (CTCTCCATGGGTTAGCTTTTTGATAGCTTC) eingesetzt. Es wurden 10 ng cDNA mit je 10 µM Primer zusammen mit einem Gemisch aus hitzestabiler DNA-Polymerase, ATP, TTP, GTP, CTP und Polymerasepuffer (vgl. z.B.: *Current Protocols in Molecular Biology*, Vol. 1, 1997, John Wiley & Sons Inc., Suppl. 37, Chapter 15), hier im Beispiel in Form des im Handel gebrauchsfertig erhältlichen "PCR-Master-Mix" der Firma Clontech, in Ansatz gebracht. Zusätzlich wurden folgende Kontrolluntersuchungen durchgeführt: 1. der vorstehend beschriebene Reaktionsansatz mit dem Plasmid pGEX-2T/pKe#165 anstelle der cDNA ("Positivkontrolle"), 2: der vorstehend beschriebene Reaktionsansatz ohne Zusatz von cDNA ("Negativkontrolle"), 3. der vorstehend beschriebene Reaktionsansatz mit GAPDH-spezifischen Primem (#302047, Stratagene; "GAPDH-Kontrolle").

Die Reaktionsprodukte der PCR-Reaktion wurden im Agarosegel elektrophoretisch aufgetrennt. **Fig. 1.A** zeigt das Ergebnis einer pKe#165-spezifischen PCR Reaktion. Es zeigt:
- Spur 1 =: DNA Molekulargewichtsmarker VII
(1209 264, Boehringer Mannheim)
- Spur 2 =: DNA Molekulargewichtsmarker VI
(1062 590, Boehringer Mannheim)
- Spur 3 =: NHEK T0 (direkt nach Ablösung),
- Spur 4 =: NHEK T2 (2 h nach Ablösung)
- Spur 5 =: NHEK T4 (4 h nach Ablösung)
- Spur 6 =: NHEK T8 (8 h nach Ablösung),
- Spur 7 =: HaCaT
- Spur 8 =: HMEC-1 (Zelllinie, in der pKe#165 nur sehr schwach nachweisbar ist)
- Spur 9 =: humane Placenta (Clontech # 7116-1)
- Spur 10 =: Negativkontrolle (keine cDNA)
- Spur 11 =: Positivkontrolle (pGEX/pKe#165-Plasmid)

Ein PCR-Produkt der erwarteten Größe von ≈ 1060 Bp wurde in den Spuren 3 - 7 nachgewiesen, d.h. pKe#165-spezifische mRNA wurde in den Keratinozytensheets (NHEK) zu den Zeitpunkten 2, 4 und 8 Stunden nach Dispase-induzierter Ablösung und ebenso in HaCaT-Zellen nachgewiesen. In Spur 8 fand sich nur eine sehr schwache Bande, was darauf hinweist, daß pKe#165-spezifische mRNA in HMEC-Zellen nur sehr schwach exprimiert ist.

**Fig. 1.B** zeigt das Ergebnis einer GAPDH-spezifischen PCR mit dem Primermix "Stratagene # 302047". Es zeigt:
- Spur 1 =: Negativkontrolle (keine cDNA)
- Spur 2 =: DNA Molekulargewichtsmarker VII
(359-8576 Bb, Boehringer Mannheim)
- Spur 3 =: NHEK T0
- Spur 4 =: NHEK T2
- Spur 5 =: NHEK T4
- Spur 6 =: NHEK T8
- Spur 7 =: HaCaT
- Spur 8 =: HMEC
- Spur 9 =: humane Placenta (Clontech #7116-1)

Diese GAPDH-spezifische PCR ("GAPDH Kontrolle") beweist, daß ein negatives PCR Ergebnis im pKe#165-spezifischen Ansatz nicht auf ein Nichtvorhandensein von cDNA zurückzuführen ist, da in allen Reaktionsansätzen von T0 bis T8 ein PCR-Produkt der erwarteten Größe von 600 Bp und in den Ansätzen mit HaCaT, HMEC und humaner Placenta GAPDH-spezifische cDNA nachweisbar war. Letzteres beweist, daß überhaupt cDNA in der Probe vorhanden war.

Die rt-PCR ermöglicht den Nachweis der pKe#165-Expression auch in Fällen, in denen der Nachweis des pKe#165-Proteins aufgrund zu niedrigen Expressionsspiegels mit immunhistologischen Methoden, dem ELISA oder mit Immunoblot-Verfahren nicht gelingt.

### Beispiel 3: Herstellung von Vektormolekülen mit der Fähigkeit zur Expression des Proteins pKe#165 in prokaryontischen bzw. eukaryontischen Zellen, sowie Produktion und Reinigung des rekombinanten pKe#165 Proteins

Zur Herstellung bzw. Expression des rekombinanten pKe#165-Proteins wurden zwei Wege beschritten. Zum einen wurde das Vektorkonstrukt pGEX-2T/pKe#165 gemäß Vektorprotokoll in **Fig. 2** für die Expression in Bakterien (E. coli DH5α) hergestellt. Zum anderen wurde das Vektorkonstrukt pcDNA3.1/ pKe#165/V5/His-TOPO gemäß Vektorprotokoll in **Fig. 3** zum Zweck der Expression in eukaryontischen Zellen (Cos-Zellen) hergestellt.

Das Vektorkonstrukt pGEX-2T/pKe#165 wurde nach Standardprotokollen für die Transformation von E. coli DH5α eingesetzt. Das pKe#165-Glutathion-S-Transferase-(GST)-Fusionsprotein wurde in Bakterien exprimiert, das bakterielle Lysat wurde im Immunoblot mit anti-GST-Antikörpern untersucht.

Das pKe#165/GST-Fusionsprotein wurde durch SDS-Polyacrylamid-Gelelektrophorese nach Standardmethoden (vgl. z.B.: Prep Cell, No. 170-2926; BioRad) aus den Bakterienlysaten gereinigt. Die Fraktionen dieser Reinigung wurden dann im Immunoblot (Western-Blot) mit anti-GST-Antikörpem (Antikörper (1) = Ziege-anti-GST [1:1500], Antikörper (2) = Kaninchen-anti-Ziege-lgG-Peroxidase [1:10000]) untersucht.

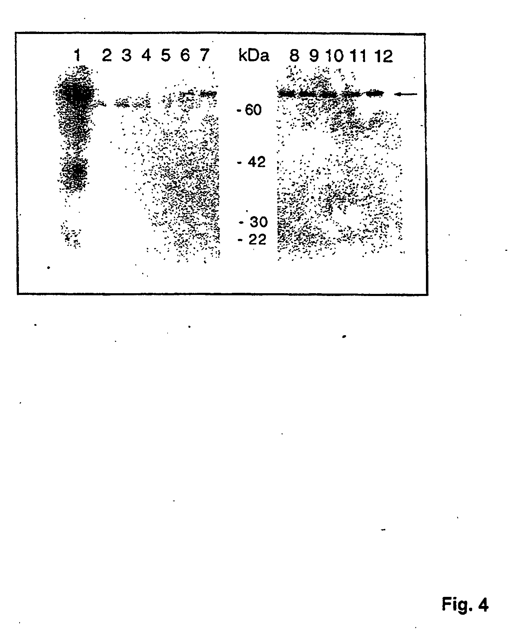

Das Produkt des Immunoblots ist in **Fig. 4** abgebildet. Es zeigt:
- Spur 1 =: das Gesamtlysat der mit pGEX-2T/pKe#165 transformierten Bakterienkultur
- Spuren 2 - 12 =: Fraktionen der Proteinreinigung (Prep Cell Reinigung)

Das pKe#165/GST-Fusionsprotein (Pfeil) hatte ein apparentes Molekulargewicht von ca. 66 kDa. Das erlaubt den Schluß, daß das 66 kDa pKe#165/GST-Fusionsprotein aus dem GST-Protein (ca. 26 kDa) und einem ca. 40 kDa großen Anteil des Proteins pKe#165 besteht.

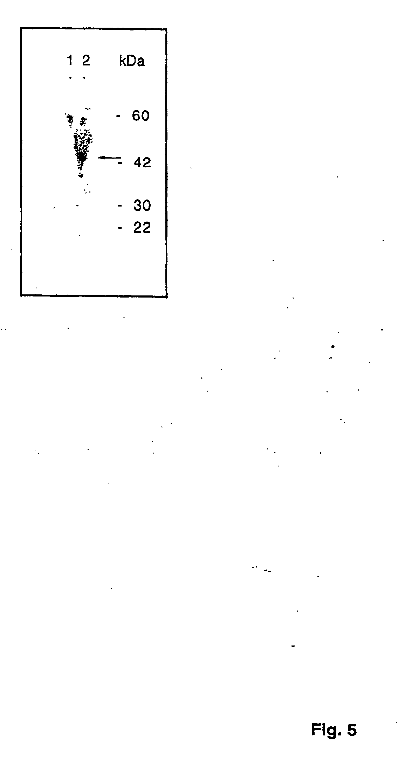

Im eukaryontischen System wurde der pcDNA3.1/pKe#165/V5/His-TOPO Vektor (**Fig. 3**) in sog. Cos-Zellen, d.h. in Zellen der im Stand der Technik allgemein bekannten Cos-Zellinie, transformiert. Die Zellen wurden nach Standardverfahren durch Behandlung mit DEAE-Dextran/Chloroquin zur Aufnahme der Plasmid-DNA gebracht. Danach wurden die transformierten Zellen drei Tage unter Standardbedingungen (37°C und 7 % CO₂) inkubiert. Die Cos-Zellen wurden lysiert und im Immunoblot (Western-Blot) unter Verwendung eines Antikörpers gegen das His-Epitop ( Antikörper (1) = Maus anti-His₅IgG1; 34660, QIAgen, Antikörper (2) = Ziege-anti-Maus-IgG-Peroxidase [1:10000]) analysiert. **Fig. 5** zeigt das Produkt des Immunoblots:
- Spur 1 =: nichttransfizierte Cos-Zellen, die mit dem Antikörper gegen das His-Epitop angefärbt wurden.
- Spur 2 =: mit dem Vektorkonstrukt pcDNA-3.1/pKe#165/V5/His-TOPO transfizierte Cos-Zellen, die mit dem Antikörper gegen das His-Epitop angefärbt wurden.

Das Ergebnis dieses Versuch belegt die Expression des ca. 50 kDa großen pKe#165/V5/His-TOPO-Fusionsproteins (Pfeil) in Cos-Zellen, die mit dem Vektorkonstrukt pcDNA3.1/pKe#165/VI/His-TOPO transfiziert wurden.

### Beispiel 4: Herstellung und Charakterisierung von Antikörpern gegen das pKe#165 Protein, sowie immunologischer Nachweis des pKe#165 Proteins mittels Immunoblot ("Westernblot"), Immunhistologie und Enzyme-Linked Immunosorbent Assay (ELISA)

Gereinigtes rekombinantes pKe#165/GST-Fusionsprotein wurde für die adjuvanzunterstützte Immunisierung von Kaninchen und Mäusen eingesetzt. Die Details des Immunisierungsverfahrens sind im Stand der Technik allgemein geläufig. Die Immunisierung von Kaninchen wurde im Kundenauftrag von der Fa. *Dr. J. Pineda Antikörper-Service* (Berlin) durchgeführt.

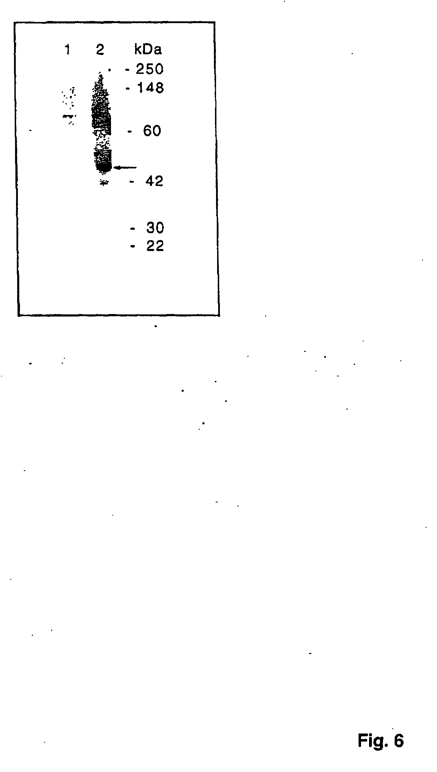

Das Maus anti-pKe#165-Serum (polyklonaler Antikörper "Maus-antipKe#165-GST") zeigte eine deutliche Immunreaktion mit dem in Cos-Zellen exprimierten rekombinanten pKe#165 Protein. Das Produkt eines entsprechenden Immunoblots (Westem-Blot mit Antikörper (1) = Maus-antipKe#165-GST [1: 1000], Antikörper (2) = Ziege-anti-Maus-IgG-Peroxidase [1: 10000]) ist in **Fig. 6** dargestellt. Es zeigt:
- Spur 1 =: mit dem "leeren" pcDNA3.1-Vektor transfizierte Cos-Zellen nach der Behandlung bzw. Immunreaktion mit Maus antipKe#165-Serum (1:1000 verdünnt)
- Spur 2 =: mit pcDNA3.1/pKe#165/V5/His-TOPO transfizierte Cos-Zellen, nach der Behandlung bzw. Immunreaktion mit Maus antipKe#165-Serum (1: 1000 verdünnt)

Der Pfeil markiert die Position des ca. 50 kDa großen pKe#165-Proteins.

**Immunhistologie:** Mit Hilfe eines Kryotoms wurden 5 µm-dicke Gefrierschnitte von Geweben aus Hautbiopsien von klinisch unauffälliger Normalhaut und von läsionaler Haut der blasenbildenden Hauterkrankung "Bullöses Pemphigoid" hergestellt. Diese wurden bei Raumtemperatur luftgetrocknet und in 100 % Azeton fixiert (anstelle von Azeton kann ebensogut auch 100 % Methanol, 100 % Ethanol oder 4 %-iges Paraformaldehyd verwendet werden). Danach wurden die Schnitte gemäß im Stand der Technik bekannter sog. "Blockierungsverfahren" behandelt, um unspezifische Bindungsstellen für den Antikörper zu blockieren. Im vorliegenden Beispielfall wurden zwei Blockierungsschritte durchgeführt: (1) eine Blockierung mit Avidin/Biotin und (2) eine Blockierung mit Normalserum. Im ersten Blockierungschritt wurde die Avidin/Biotin-Blockierung unter Verwendung des Avidin/Biotin-Blockierungskits der Firma Vector-Laboratories nach Herstellervorschrift eingesetzt, d.h. es wurde bei Raumtemperatur zunächst 15 Minuten mit der Avidin-Fertiglösung und nachfolgend 15 Minuten mit der mit der Biotin-Fertiglösung inkubiert. Anschließend wurden die Schnitte mit 10 Vol-% Normalserum in PBS für 15 Minuten bei Raumtemperatur inkubiert (Normalserum der Spezies, aus der der Zweitantikörper stammt, hier Ziege-Normalserum, PBS = Phosphate buffered saline = Phosphat-gepufferte Kochsalzlösung, pH 7,2- 7,4).

Aus den Immunseren wurde die IgG-Fraktion nach Standardverfahren mittels Ammoniumsulfatfällung isoliert. Die resultierenden IgG-Präparationen werden im folgenden als "anti-pKe#165 IgG" bezeichnet.

Im Anschluß an die Blockierung wurden die Schnitte in PBS mit einem Gehalt an Kaninchen "anti-pKe#165 IgG" für 1 Stunde bei Raumtemperatur inkubiert. Zur Entfernung des nichtgebundenen Antikörpers wurden die Schnitte anschließend in PBS mit einem Gehalt an 0,2 % (Gewicht/Volumen) bovinem Serumalbumin gewaschen. Es folgt die Inkubation mit einem beispielsweise Biotin-markierten und gegen Kaninchen-IgG-gerichteten Antikörper aus der Ziege (1:500 verdünnt in PBS/ 0,2% BSA; 30 Minuten bei Raumtemperatur), ein weiterer Waschschritt, sowie die Aufbringung eines mit dem Fluoreszenzfarbstoff Cy3-markierten Streptavidins (1 : 1000 in PBS/0,2 % BSA verdünnt). Anstelle von Cy3 kann auch ein anderer Fluoreszenzfarbstoff zur Markierung des Streptavidins verwendet werden, z.B. FITC. Nach einem letzten Waschschritt wurden die Schnitte mit Eindeckmedium, z.B. Elvanol oder Histogel, eingedeckt und im Fluoreszenzmikroskop untersucht und ausgewertet.

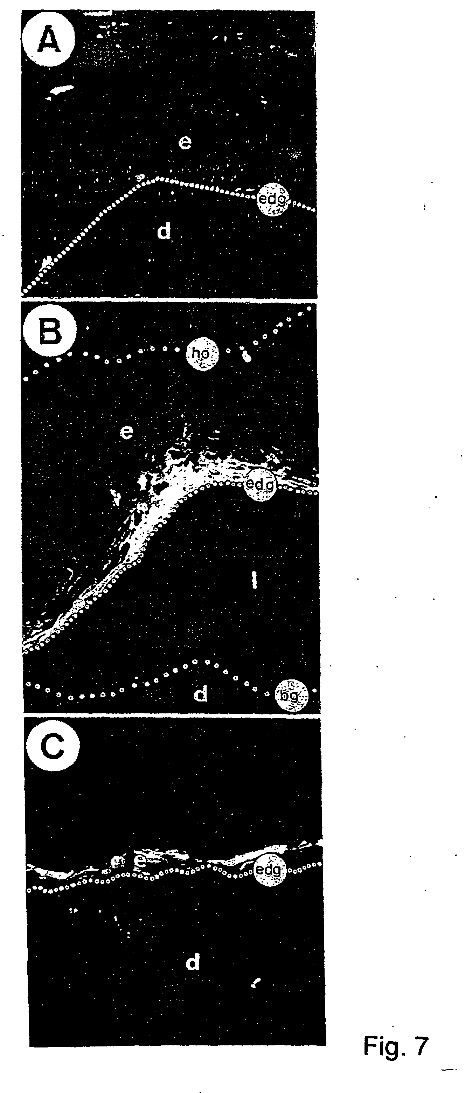

In Fig. 7 sind die Ergebnisse eines derart durchgeführten Immunfluoreszenztests dargestellt. In dieser Darstellung bedeuten: e=Epidermis, d=Dermis, edg=epidermale Grenzzone, ho=Hautoberfläche, bg=Blasengrund. Das Kaninchen anti-pKe#165 IgG zeigt auf Normalhautschnitten eine schwache Immunfärbung (Fig. 7.A). In läsionaler Haut von bullösem Pemphigoid zeigt das anti-pKe#165 IgG eine deutliche Fluoreszenz im Bereich der Keratinozyten, die in die Läsion einbezogen sind (Fig. 7.B). Insbesondere findet sich eine deutliche Immunfluoreszenz im Bereich von Keratinozyten, die den läsionalen Bereich reepithelisieren (Fig. 7.C). Dieses Ergebnis weist darauf hin, daß in Keratinozyten normaler Haut wenig Protein pKe#165 vorhanden ist, während in Keratinozyten, die in die epidermalen Läsionen einbezogen sind, eine vermehrte Expression dieses Proteins pKe165 stattfindet.

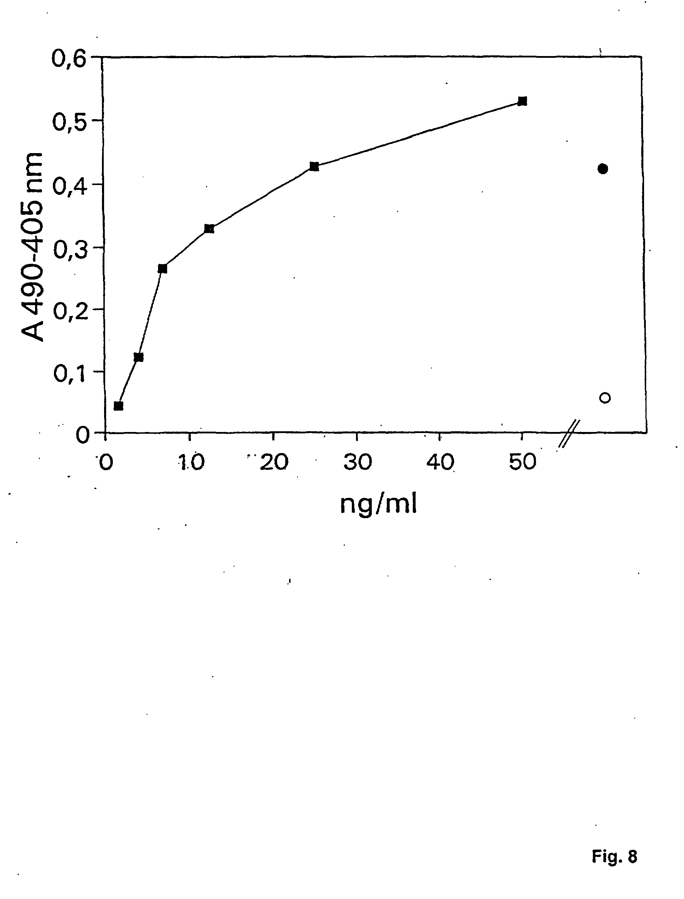

**Enzyme-linked Immunosorbent Assay (ELISA):** Zur Quantifizierung des pKe#165-Proteins in komplexen Lösungen wurde ein sog. "Sandwich"-ELISA (**Fig. 8**) durchgeführt. Hierzu wurde eine Mikrotiterplatte mit einem gegen pKe#165 gerichteten Antikörper (z.B. Kaninchen anti-pKe#165 IgG, 1µg/Vertiefung) beschichtet. Dann wurden die noch verbliebenen unspezifischen Bindungsstellen der Mikrotiterplatte durch Behandlung mit 0,1 Gewichts-% Gelatine in phospatgepufferter Kochsalzlösung ("PBS/Gelatine") blockiert. Anschließend wurde die Mikrotiterplatte mit unterschiedlichen Konzentrationen des pKe#165-GST-Fusionsproteins als Kalibrator, bzw. mit Verdünnungen unbekannter Proben (in denen die pKe#165-Konzentration festgestellt werden sollte) in Ansatz gebracht. Nach einem Waschschritt mit 0,05 Volumen-% Tween-20 in PBS (PBS/Tween) wurde die Platte mit einer IgG Präparation aus einer zweiten Spezies (z.B. mit Maus anti-pKe#165 IgG) inkubiert (z.B. eine Stunde unter Schütteln bei Raumtemperatur). Nach einem weiteren Waschschritt mit PBS/Tween wurde die Platte mit einer Peroxidase-markierten kommerziellen Kaninchen anti Maus-IgG Antikörper-Präparation inkubiert (z.B. Fc-spez. Fab₂-POX von Dianova GmbH, Hamburg). "Peroxidase" steht hier stellvertretend für praktisch jede beliebige Markierung des Antikörpers, z.B. mit Enzymen, Fluoreszenzmolekülen oder Lumineszenzmolekülen. Nach einem weiteren Waschschritt zur Entfernung ungebundener enzymmarkierter Antikörper wurde das farblose Peroxidase-Substrat Ortho-Phenylendiamin zugesetzt, welches durch die Peroxidase-Aktivität in ein farbiges Produkt umgewandelt wird. Die Quantifizierung der Farbbildung erfolgt in einem Mikrotiterplattenphotometer bei 490 nm gegen 405 nm (Ordinate).

Das Ergebnis des Versuchs ist in **Fig. 8** dargestellt. Es zeigt, daß die Farbkonzentration (angegeben als Absorption in der Ordinate) der Menge des eingesetzten pKe#165-Proteins (= des "Kalibrators", in der Abszisse dargestellt) proportional ist. Um die Funktionalität des Testsystems zu demonstrieren, wurden gleichzeitig Lysate von zwei verschiedenen Cos-Transfektanten-Ansätzen getestet, die sich in der Expression von pKe#165 unterscheiden. Die Cos-Zellen des einen Ansatzes wurden mit dem Vektorkonstrukt pcDNA3.1/pKe#165 (Markierung: geschlossener Kreis) und die des anderen Ansatzes mit dem pcDNA3.1 Vektor ohne Insert (Markierung: offener Kreis) transfiziert.

Zellen dieser Transfektanten-Ansätze wurden nach Standardverfahren unter Verwendung des Detergenzes Triton X-100 lysiert. Diese Lysate wurden in einer 1:10-Verdünnung in PBS/Tween 20 im ELISA getestet. Lysate des "Cos pKe#165"-Transfektanten-Ansatzes zeigten eine positive Reaktion zeigen. Unter Berücksichtigung der Kalibratordaten wurde eine Konzentration von ca. 25 ng pKe#165/ 10⁶ Cos pKe#165-Zellen festgestellt. Bei den Lysaten der Kontroll-Transfektanten-Ansätze "Cos" konnte kein Protein pKe#165 nachgewiesen werden. Durch den Einsatz dieses Testverfahrens ist folglich eine Quantifizierung einer unbekannten Menge des Proteins pKe#165 in einer Probe möglich.

Die Substanz Ortho-Phenylendiamin steht hier stellvertretend für jedes beliebige Peroxidase-Substrat, das infolge der Peroxidase-Aktivität seine Farbe nachweisbar verändert. Anstelle der hier beispielhaft verwendeten polyklonalen Antikörper können ebensogut monoklonale Antikörper, die gegen das Protein pKe#165 gerichtet sind, eingesetzt werden. Anstatt des indirekten Ansatzes über einen markierten speziesspezifischen anti-IgG Antikörper kann auch die Durchführung mit einem direkt markierten antipKe#165-Antikörper erfolgen.

### Beispiel 5: Beeinflussung von Keratinozyten durch pKe#165-spezifische Oligonukleotide

Antisense-Oligonukleotide werden von Zellen, auch Keratinozyten, aufgenommen (vgl.: *G. Hartmann et al. 1998: Antisense-Oligonukleotide, Deutsches Ärzteblatt 95, Heft 24, C1115 - C1119*). Sie binden in spezifischer Weise an die in der Zelle vorliegende mRNA und hemmen deren Translation und damit die Expression des entsprechenden Proteins (vgl.: *Y.-S. Lee et al. 1997, Definition by specific antisense oligonucleotides of a role for protein kinase Cα in expression of differentiation markers in normal and neoplastic mouse epidermal keratinocytes, Molecular Carcinogenesis 18: 44-53*). Geeignete Antisense-Oligonukleotide wurden anhand der pKe#165-spezifischen Nukleotidsequenz (**SEQ ID NO:1**) hergestellt. Sie wurden mit geeignetem Puffermedium (sog. "Oligopuffer") auf eine Konzentration von 100 µM eingestellt. HaCaT-Zellen wurden bei 37°C und 7 % CO₂ bis zu einer Konfluenz von 70 - 80 % kultiviert. Die Zellen wurden abtrypsiniert (10 Minuten 0,2 Gewichts-% EDTA, 0,1 Gewichts-% Trypsin, 5-10 Minuten) und auf eine Konzentration von 25 000 Zellen/ml eingestellt. Pro Vertiefung einer Mikrotiter-Kulturplatte (96 Vertiefungen) wurden 100 µl Zellsuspension (entspricht 2500 Zellen) einpipepttiert. Die Zellen wurden für 1 Stunde unter den vorgenannten Kultivierungsbedingungen inkubiert, danach erfolgte die Zugabe des Antisense-Oligonukleotids (2 µl einer 100 µM-Lösung) und eine weitere Inkubation von 24 - 48 Stunden. Als Negativkontrolle dienten Zellansätze, denen Oligonukleotide mit der gleichen Basenverteilung aber zufällig ausgewählter Sequenz zugegeben wurden.

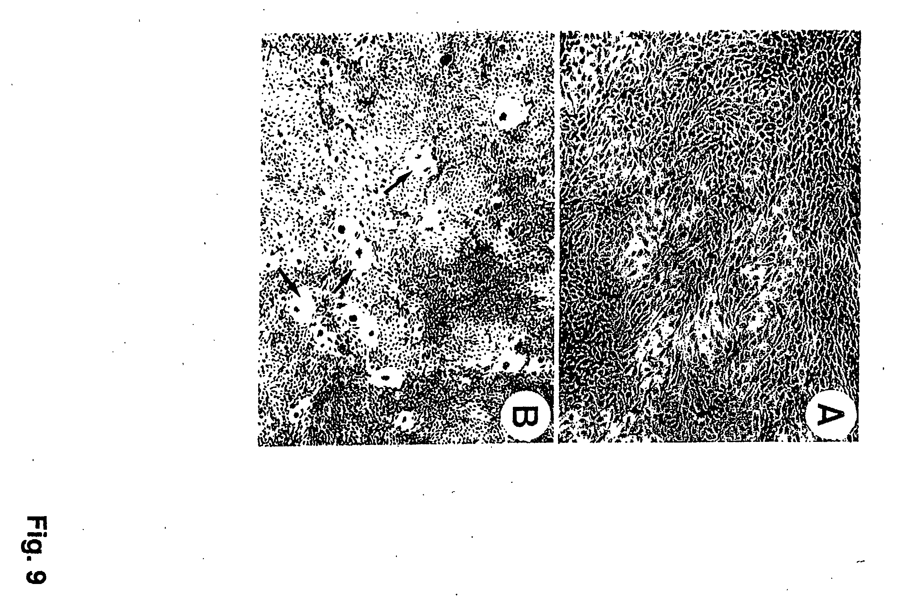

Die solcherart behandelten Zellen wurden mit Hilfe eines Mikroskops hinsichtlich phänotypischer Veränderungen untersucht. Das Ergebnis der mikroskopischen Analyse ist in **Fig. 9** dargestellt: **Fig. 9.A** zeigt HaCaT-Zellen, die mit Kontroll-Oligonukleotiden behandelt worden sind, **Fig. 9.B** zeigt HaCaT-Zellen, die mit pKe#165-spezifischen Antisense-Oligonukleotiden behandelt worden sind.

Die mikroskopischen Untersuchungen zeigten, daß in den mit Antisense-Oligonukleotiden behandelten HaCaT-Kulturen stark vergrößerte Zellen auftraten (**Fig. 9.B**, Pfeile), die in den mit Kontroll-Oligonukleotiden behandelten Kulturen nicht zu finden waren. Diese großen Zellen entsprechen in ihrer Morphologie differenzierten Keratinozyten. Der Befund weist darauf hin, daß mit pKe#165-spezifischen Antisense-Oligonukleotiden behandelte Zellen eine vermehrte Tendenz zur Differenzierung aufweisen.

## Patentansprüche

1. Isoliertes Polypeptid, das die im Sequenzprotokoll SEQ ID NO: 2 dargestellte Aminosäuresequenz aufweist, natürlicherweise in humanen epidermalen Keratinozyten vorkommt und im aktivierten, durch eine erhöhte Expression der Aktivierungsmarker uPA und uPA-R gekennzeichneten Zustand dieser Keratinozyten aufreguliert, nämlich verstärkt exprimiert wird.

2. Isolierte Nukleinsäure, die entweder die im Sequenzprotokoll SEQ ID NO: 1 dargestellte Nukleotidsequenz oder eine ganz oder teilweise mit dieser unter stringenten Bedingungen hybridisierende Nukleotidsequenz aufweist, und die ein Polypeptid gemäß Anspruch 1 codiert.

3. Isolierte Nukleinsäure nach Anspruch 2, **dadurch gekennzeichnet, daß** diese Nukleinsäure aus einer natürlichen, synthetischen oder halbsynthetischen Quelle gewonnen ist.

4. Isolierte Nukleinsäure nach Anspruch 2 oder 3 **dadurch gekennzeichnet, daß** diese Nukleinsäure eine cDNA ist.

5. Rekombinantes DNS-Vektormolekül, das eine Nukleinsäure nach einem der Ansprüche 2 bis 4 umfaßt, und das die Fähigkeit zur Expression eines Polypeptids gemäß Anspruch 1 in einer prokaryontischen oder eukaryontischen Zelle aufweist.

6. Rekombinantes DNS-Vektormolekül nach Anspruch 5, **dadurch gekennzeichnet, daß** das Vektormolekül ein Abkömmling, nämlich ein Vektorkonstrukt des Plasmids pUEX-1 oder des Plasmids pGEX-2T oder des Plasmids pcDNA3.1 ist.

7. Rekombinantes DNS-Vektormolekül nach Anspruch 6, **dadurch gekennzeichnet, daß** das Vektormolekül ein Konstrukt gemäß Vektorprotokoll in Fig. 2 oder gemäß Vektorprotokoll in Fig. 3 ist, wobei diese Vektorprotokolle Fig.2 und Fig.3 Bestandteile dieses Anspruchs sind.

8. Transformierte Wirtszelle, die ein Vektormolekül nach einem der Ansprüche 5 bis 7 enthält.

9. Transformierte Wirtszelle, die eine Nukleinsäure nach einem der Ansprüche 2 bis 4 enthält, welche mit einem aktivierbaren Promotor gekoppelt ist, und **dadurch gekennzeichnet ist, daß** der Promotor der Zytokeratin-14-Promotor und die Wirtszelle ein Keratinozyt ist, oder daß der Promotor der CMV-Promotor und die Wirtszelle eine Cos-Zelle ist.

10. Verwendung eines Polypeptids nach Anspruch 1 zur Herstellung eines isolierten Antikörpers gegen dieses Polypeptid.

11. Antikörper, der spezifisch mit einem Polypeptid gemäß Anspruch 1 reagiert.

12. Reagenz zum indirekten Nachweis des in humanen Keratinozyten vorkommenden und in aktivierten Keratinozyten verstärkt exprimierten Proteins mit der Aminosäuresequenz gemäß SEQ ID NO: 2, **dadurch gekennzeichnet, daß** das Reagenz wenigstens eine Nukleinsäure nach einem der Ansprüche 2 bis 4 umfaßt.

13. Verwendung eines Polypeptids nach Anspruch 1 oder einer Nukleinsäure nach Anspruch 2 in einem Screeningverfahren zur Identifizierung von medizinisch, kosmetisch oder pharmakologisch einsetzbaren Substanzen, die an das Polypeptid bzw. die Nukleinsäure binden und dadurch dessen/deren Funktion und/oder Expression beeinflussen.

## Claims

1. Isolated polypeptide which comprises the amino acid sequence shown in the sequence protocol SEQ ID NO: 2, which naturally occurs in human epidermal keratinocytes and which is upregulated, i.e. increasingly expressed, in the activated stated of the keratinocytes marked by increased expression of the activation markers uPA and uPA-R.

2. Isolated nucleic acid which comprises either the nucleotide sequence shown in the sequence protocol SEQ ID NO: 1 or a nucleotide sequence which hybridizes either entirely or partially with the same under stringent conditions and which codes a polypeptide according to Claim 1.

3. Isolated nucleic acid according to Claim 2, **characterized in that** this nucleic acid is obtained from a natural, synthetic or semisynthetic source.

4. Isolated nucleic acid according to Claim 2 or 3, **characterized in that** this nucleic acid is a cDNA.

5. Recombinant DNA vector molecule comprising a nucleic acid according to one of Claims 2 through 4 and being capable of expressing a polypeptide according to Claim 1 in a prokaryotic or eukaryotic cell.

6. Recombinant DNA vector molecule according to Claim 5, **characterized in that** the vector molecule is a derivative, i.e. a vector construct of the plasmid pUEX-1 or of the plasmid pGEX-2T or of the plasmid pcDNA3.1.

7. Recombinant DNA vector molecule according to Claim 6, **characterized in that** the vector molecule is a construct according to the vector protocol in Fig. 2 or according to the vector protocol in Fig. 3, wherein these vector protocols Fig.2 and Fig.3 represent an integral part of this present Claim.

8. Transformed host cell containing a vector molecule according to one of Claims 5 through 7.

9. Transformed host cell containing a nucleic acid according to one of Claims 2 through 4 which is coupled with an activatable promoter and being **characterized in that** the promoter is the cytokeratin-14 promoter and the host cell a keratinocyte or **in that** the promoter is the CMV promoter and the host cell is a Cos cell.

10. Use of a polypeptide according to Claim 1 for manufacturing of an isolated antibody against such polypeptide.

11. Antibody which reacts specifically with a polypeptide according to Claim 1.

12. Reagent for indirect detection of the protein occurring in the human keratinocytes and being increasingly expressed in activated keratinocytes and having the amino acid sequence according to SEQ ID NO: 2, **characterized in that**
the reagent comprises at least one nucleic acid according to one of Claims 2 through 4.

13. Use of a polypeptide according to Claim 1 or of a nucleic acid according to Claim 2 in a screening procedure to identify substances which can be used for medical, cosmetic or pharmacological purposes and which bind to the polypeptide resp. nucleic acid and thereby impact its/their function and/or expression.

## Revendications

1. Polypeptide isolé, qui présente la séquence d'acide aminé présentée dans le protocole de séquence SEQ ID NO :2, qui apparaît naturellement dans les kératinozytes épidermaux humains et qui est régulé, c'est-à-dire exprimé de manière accrue, à l'état activé, **caractérisé par** une expression accrue des marqueurs d'activation uPA et uPA-R de ces kératinozytes.

2. Acide nucléique isolé, qui présente soit la séquence de nucléotide présentée dans le protocole de séquence SEQ ID NO :1, soit une séquence nucléotide hybridisant totalement ou partiellement avec celle-ci dans des conditions rigoureuses, et qui code un polypeptide selon la revendication 1.

3. Acide nucléique isolé selon la revendication 2, **caractérisé en ce que** cet acide nucléique est obtenu à partir d'une source naturelle, synthétique ou semi-synthétique.

4. Acide nucléique isolé selon la revendication 2 ou 3, **caractérisé en ce que** cet acide nucléique est un cDNA.

5. Molécule vectrice d'ADN de recombinaison qui contient un acide nucléique selon une des revendications 2 à 4, et qui présente la faculté d'expression d'un polypeptide selon la revendication 1 dans une cellule prokaryontique ou eukaryontique.

6. Molécule vectrice d'ADN de recombinaison selon la revendication 5, **caractérisée en ce que** la molécule vectrice est une descendante, à savoir une création vectrice du plasmide pUEX-1 ou du plasmide pGEX-2T ou du plasmide pcDNA3.1.

7. Molécule vectrice d'ADN de recombinaison selon la revendication 6, **caractérisée en ce que** la molécule vectrice est une création selon le protocole vecteur en figure 2 ou selon le protocole vecteur en figure 3, ces protocoles vecteurs des figures 2 et 3 faisant partie intégrante de cette revendication.

8. Cellule-hôte transformée, qui contient une molécule vectrice selon une des revendications 5 à 7.

9. Cellule-hôte transformée, qui contient un acide nucléique selon une des revendications 2 à 4, qui est coupleè à un promoteur activable, et **caractérisée en ce que** le promoteur est le promoteur de zytokératine 14 et que la cellule-hôte est un kératinozyte, ou que le promoteur est le promoteur de CMV et la cellule-hôte une cellule Cos.

10. Utilisation d'un polypeptide selon la revendication 1 pour la fabrication d'un anticorps isolé contre ce polypeptide.

11. Anticorps qui réagit spécifiquement avec un polypeptide selon la revendication 1.

12. Réactif fournissant la preuve indirecte de la protéine apparaissant dans les kératinozytes humains et exprimée de manière accrue dans les kératinozytes activés avec la séquence d'acide aminé selon SEQ ID NO :2, **caractérisée en ce que** le réactif comprend au moins un acide nucléique selon une des revendications 2 à 4.

13. Utilisation d'un polypeptide selon la revendication 1 ou d'un acide nucléique selon la revendication 2 dans un procédé de criblage pour l'identification de substances utilisables au niveau médical, cosmétique ou pharmacologique, substances qui se lient au polypeptide ou à l'acide nucléique et influent ainsi sur la fonction et/ou l'expression de celui-ci.
